# EUROPEAN PATENT APPLICATION

(11) **EP 3 918 910 A1**
(43) Date of publication of application: **08.12.2021**
(21) Application number: 20178623.3
(22) Date of filing: 05.06.2020
(51) Int. Cl.: A01H 1/04, C12N 15/82, C12Q 1/6895

(54) **MARKER GENERATION BY RANDOM MUTAGENESIS IN PLANTS**

(71) Applicant: KWS SAAT SE & Co. KGaA, 37574 Einbeck (DE)
(72) Inventor: Kloiber-Maitz, Monika, 37574 Einbeck (DE); Reuscher, Stefan, 37574 Einbeck (DE)

(57) **Abstract**

The present invention relates to a method for introducing genetic markers in plants by random mutagenesis, in particular linked to a genomic region of interest. The present invention further relates to a method generating such plants by random mutagenesis as well as a method for identifying such plants by identification of the genetic marker linked to the genomic region of interest.

## Description

### FIELD OF THE INVENTION

The invention relates to the field of plant breeding, and in particular to marker assisted selection of traits.

### BACKGROUND OF THE INVENTION

Agricultural breeding processes almost always invariantly aim at optimizing plant performance. In particular, plant breeding schemes developed to increase crop yield are of prime importance. In addition, other agronomically relevant traits, such as for instance disease resistance, stress tolerance, or quality, are subject to plant breeding efforts.

Phenotypic screening and selection of plants carrying the relevant trait of interest is often cumbersome and may take a long time to produce results, as often plants need to be grown to maturity in order to evaluate the trait. In this context, marker assisted selection is a valuable tool which may significantly speed up the process. Indeed, identification of markers closely linked to the trait of interest allows selection of desired plants at a very early stage. Furthermore, in order to actively select against linkage drag, suitable markers need to be in close proximity to a trait of interest, such that recombination between a trait of interest and its flanking regions can be adequately monitored.

One major hurdle in marker assisted selection is in fact the identification of relevant markers. As the occasion may be, no markers closely linked (or close enough linked) to the trait of interest may be able to be identified. This can for instance be the case if the trait of interest is encoded in a genomic region of low polymorphism. Alternatively, markers linked to a trait of interest may ultimately not be proven to be unique, such that these do not invariantly allow for identification of the trait of interest. These problems can be significant, in particular in connection with complex traits, such as encoded by quantitative trait loci (QTL) or highly polymorphic traits. Also, if the boundaries of for instance a QTL are not definitely determined, or not appropriately fine-mapped, linkage drag can become a significant problem.

It is therefore an objective of the present invention to address one or more of the shortcomings of the prior art.

### SUMMARY OF THE INVENTION

The present invention relates to the generation and introduction of (genetic) markers, in particular *de novo* generation and introduction of (genetic) markers in plants, plant parts or plant populations. Markers are advantageously introduced by random mutagenesis. Suitable markers are obtained which are linked with a genomic region of interest, such as a gene or QTL or generally any locus conferring a trait on a plant or plant part or plant population. Preferably, the markers are in (local) regions of low polymorphism (surrounding the genomic region of interest).

The present invention further relates to the generation of plants by introducing (genetic) markers, in particular *de novo* generation and introduction of (genetic) markers in plants, plant parts or plant populations, in particular by random mutagenesis, and in particular markers linked with a genomic region of interest.

The invention further relates to the identification of such (genetic) markers as well as the identification of plants, plant parts, or plant populations comprising such (genetic) markers, in particular markers linked with a genomic region of interest.

The present invention also relates to plants, plant parts, populations, or progeny thereof which are generated or identified according to the methods or uses of the invention as described herein.

The present invention also relates to mutations or genetic markers which are generated or identified according to the methods or uses of the invention as described herein. The present invention also relates to (isolated) polynucleic acids comprising mutations or genetic markers which are generated or identified according to the methods or uses of the invention as described herein, the complement, or reverse complement thereof. The present invention also relates to (isolated) polynucleic acids specifically hybridizing with polynucleotide sequences comprising mutations or genetic markers which are generated or identified according to the methods or uses of the invention as described herein, the complement, or reverse complement thereof. Such polynucleic acids may in particular be primers or probes, such as primers or probes suitable for detecting the mutations or genetic markers as described herein.

One of the advantages of the present invention, and in particular of the use of random mutagenesis in the methods provided herein, lies in the fact that no prior knowledge about exact genomic location, and in particular of the genomic boundaries, of the trait of interest is needed. Indeed, mutations can be introduced at regular intervals, which allows to progressively narrow down the genomic region in order to more exactly pinpoint the location of the locus conferring the trait. Accordingly, the present invention advantageously can be used for fine-mapping a locus conferring at trait. The present invention therefore also advantageously allows to minimize linkage drag of unwanted or deleterious genes flanking the locus conferring the trait of interest.

Moreover, the present invention does not involve transgenesis, which may be an advantage for instance in regions or countries where transgenic plants cannot or may not be used.

The present invention is in particular captured by any one or any combination of one or more of the below numbered statements 1 to 57, as such or combined with any other statement and/or embodiments provided herein.
1. Method for introducing one or more mutation or genetic marker (linked with a genomic region of interest) in a plant or plant part, comprising subjecting a plant or plant part (population) to random mutagenesis and selecting a plant or plant part or progeny thereof comprising a mutation or genetic marker (introduced by random mutagenesis and/or linked with a genomic region of interest).
2. Method for generating a plant or plant part (comprising a genomic region of interest), comprising subjecting a plant or plant part (population) to random mutagenesis and selecting a plant or plant part or progeny thereof comprising a mutation or genetic marker (introduced by random mutagenesis and/or linked with a genomic region of interest).
3. Method for identifying one or more mutation or genetic marker (linked with a genomic region of interest) in a plant or plant part, comprising identifying or screening for the presence of one or more mutation or genetic marker introduced in a plant or plant part (population) by random mutagenesis (and linked with a genomic region of interest).
4. Method for identifying a plant or plant part (comprising a genomic region of interest), comprising identifying or screening for the presence of one or more mutation or genetic marker introduced in a plant or plant part (population) by random mutagenesis (and linked with a genomic region of interest).
5. Method for fine mapping a genomic region of interest in a plant or plant part, comprising screening for the presence of one or more mutation or genetic marker introduced in a plant or plant part (population) by random mutagenesis (and linked with a genomic region of interest).
6. Use of random mutagenesis for introducing one or more mutation or genetic marker (introduced by random mutagenesis) linked with a genomic region of interest in a plant or plant part (population).
7. Use of random mutagenesis for generating a plant or plant part (population) comprising one or more mutation or genetic marker (introduced by random mutagenesis) linked with a genomic region of interest.
8. Use of a randomly mutated plant or plant part (population), or progeny thereof, for identifying one or more mutation or genetic marker (introduced by random mutagenesis) linked with a genomic region of interest.
9. Use of a randomly mutated plant or plant part (population), or progeny thereof, for identifying a plant or plant part comprising one or more mutation or genetic marker (introduced by random mutagenesis) linked with a genomic region of interest.
10. Use of random mutagenesis for fine mapping a genomic region of interest in a plant or plant part (population).
11. The method or use according to any of statements 1 to 10, wherein said one or more mutation or genetic marker is unique in said plant or plant part (population).
12. The method or use according to any of statements 1 to 11, wherein said one or more mutation or genetic marker is not naturally present in said plant or plant part (population).
13. The method or use according to any of statements 1 to 12, wherein said one or more mutation or genetic marker is selectable.
14. The method or use according to any of statements 1 to 13, wherein said one or more mutation or genetic marker does not cause a phenotypic effect.
15. The method or use according to any of statements 1 to 14, wherein said one or more mutation or genetic marker is not a functional mutation.
16. The method or use according to any of statements 1 to 15, wherein said one or more mutation or genetic marker comprises one or more of: one or more insertion, one or more deletion, or one or more SNP, preferably one or more SNP.
17. The method or use according to any of statements 1 to 16, wherein said one or more mutation or genetic marker is flanking said genomic region of interest.
18. The method or use according to any of statements 1 to 17, wherein said one or more mutation or genetic marker is located within said genomic region of interest.
19. The method or use according to any of statements 1 to 18, wherein said one or more mutation or genetic marker comprises two mutations or genetic markers.
20. The method or use according to any of statements 1 to 19, wherein said one or more mutation or genetic marker comprises one mutation or genetic marker 5' of said genomic region of interest and one mutation or genetic marker 3' of said genomic region of interest.
21. The method or use according to any of statements 1 to 20, wherein said one or more mutation or genetic marker is located within 20 cM, preferably 10 cM, more preferably 2 cM of said genomic region of interest.
22. The method or use according to any of statements 1 to 21, wherein the LOD score of said one or more mutation or genetic marker and said genomic region of interest is at least 3, preferably at least 4.
23. The method or use according to any of statements 1 to 22, wherein said one or more mutation or genetic marker is a mutation or genetic marker allele and/or wherein said genomic region of interest is a genomic region of interest allele.
24. The method or use according to any of statements 1 to 23, wherein said one or more mutation or genetic marker is homozygous.
25. The method or use according to any of statements 1 to 23, wherein said one or more mutation or genetic marker is heterozygous.
26. The method or use according to any of statements 1 to 25, wherein said genomic region of interest comprises a locus of a trait of interest.
27. The method or use according to any of statements 1 to 26, wherein said genomic region of interest comprises a gene or a QTL.
28. The method or use according to any of statements 1 to 27, wherein said genomic region of interest confers a trait upon said plant or plant part (population).
29. The method or use according to any of statements 1 to 28, wherein said genomic region of interest is in a (local) area of low polymorphism.
30. The method or use according to statement 29, wherein said area of low polymorphism comprises in a population of said plant or plant part (on average) less than 2 polymorphisms up to 1 Mb up- and/or downstream of said genomic region of interest.
31. The method or use according to any of statements 29 to 30, wherein said area of low polymorphism comprises, in a population of said plant or plant part, (on average) less than 2 unique polymorphisms up to 1 Mb up- and/or downstream of said genomic region of interest.
32. The method or use according to any of statements 29 to 31, wherein said area of low polymorphism comprises in a population of said plant or plant part (on average) less than 2 unique polymorphisms linked with said genomic region of interest (up to 1 Mb up- and/or downstream of said genomic region of interest).
33. The method or use according to any of statements 29 to 32, wherein said area of low polymorphism comprises in a population of said plant or plant part (on average) less than 2 polymorphisms within 2 cM of said genomic region of interest.
34. The method or use according to any of statements 29 to 33, wherein said area of low polymorphism comprises in a population of said plant or plant part (on average) less than 2 unique polymorphisms within 2 cM of said genomic region of interest.
35. The method or use according to any of statements 29 to 34, wherein said area of low polymorphism comprises in a population of said plant or plant part (on average) less than 2 polymorphisms having an LOD score of at least 3 with said genomic region of interest.
36. The method or use according to any of statements 29 to 34, wherein said area of low polymorphism comprises in a population of said plant or plant part (on average) less than 2 unique polymorphisms having an LOD score of at least 3 with said genomic region of interest.
37. The method or use according to any of statements 29 to 36, wherein said level/degree of polymorphism (θₛ) is at most 5% (within 10 Mb up- and/or downstream of said genomic region of interest).
38. The method or use according to any of statements 1 to 37, wherein said random mutagenesis comprises irradiation.
39. The method or use according to any of statements 1 to 8, wherein said random mutagenesis comprises mutagenesis with X-rays, gamma rays, or UV.
40. The method or use according to any of statements 1 to 39, wherein said random mutagenesis comprises chemical mutagenesis.
41. The method or use according to any of statements 1 to 40, wherein said random mutagenesis comprises mutagenesis with EMS, ENU, or DMS.
42. The method or use according to any of statements 1 to 41, wherein said random mutagenesis comprises single nucleotide mutagenesis.
43. The method or use according to any of statements 1 to 42, wherein said random mutagenesis results in an average number of mutations ranging from 1 to 20 mutations per 1 Mb.
44. The method or use according to any of statements 1 to 43, wherein said population is a plant line or strain.
45. The method or use according to any of statements 1 to 44, wherein said population is an inbred plant line or strain.
46. The method or use according to any of statements 1 to 44, wherein said population is an outbred plant line or strain.
47. The method or use according to any of statements 1 to 46, wherein said population of plants has a (average) degree of homozygosity of at least 50%.
48. The method or use according to any of statements 1 to 47, comprising subjecting said plant, plant part, or plant population to random mutagenesis.
49. The method or use according to any of statements 1 to 48, comprising selecting a plant or plant part comprising the genomic region of interest.
50. The method or use according to any of statements 1 to 49, comprising screening for the presence of/identifying a mutation or genetic marker introduced by random mutagenesis.
51. The method or use according to any of statements 1 to 50, comprising sequencing at least the 5' and/or 3' flanking region of said genomic region of interest.
52. The method or use according to any of statements 1 to 51, comprising sequencing at least 1 Mb of the 5' and/or 3' flanking region of said genomic region of interest.
53. The method or use according to any of statements 1 to 52, comprising stabilizing or fixing the genomic region of interest in a plant population.
54. The method or use according to any of statements 1 to 53, comprising backcrossing said plant.
55. The method or use according to any of statements 1 to 54, comprising selfing said plant.
56. The method or use according to any of statements 1 to 55, comprising generating a double haploid plant.
57. The method or use according to any of statements 1 to 56, comprising:
   - Subjecting a plant or plant part population comprising a genomic region of interest to random mutagenesis;
   - Optionally propagating said plant or plant part population and selecting a plant or plant part comprising said genomic region of interest;
   - Sequencing at least the 5' and/or 3' flanking region of said genomic region of interest;
   - Identifying (and/or selecting) a plant or plant part comprising one or more mutation introduced in said 5' and/or 3' flanking region.

### BRIEF DESCRIPTION OF THE FIGURES

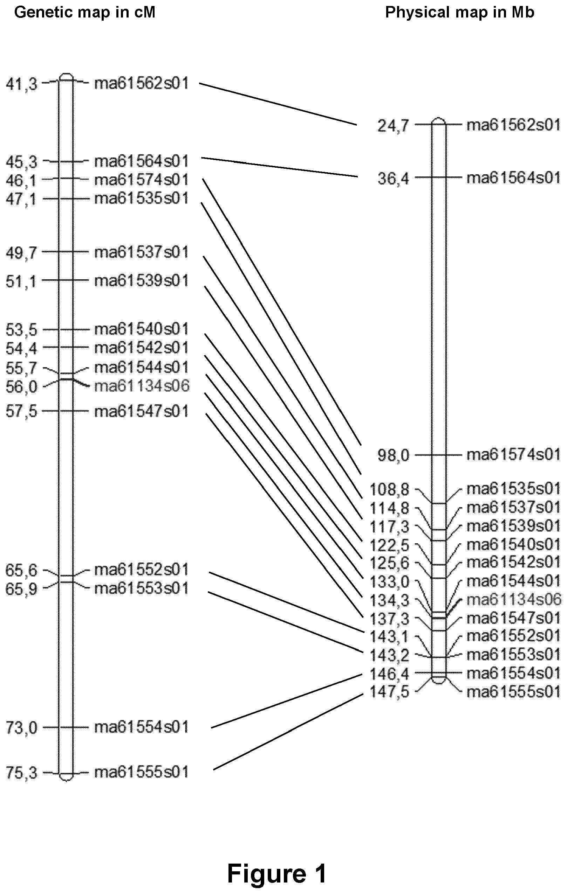

**Figure 1****:** Genetic (left) and physical map (right) of a region of chromosome 9 of *Zea mays* line PH207 indicating markers introduced by random mutagenesis. ma61134s06 represents a functional marker (mutation) in the cytochrome P450 flavonoid 3',5'-hydroxylase (F35H) target gene. The genetic positions are projected using common markers between physical and genetic map.

### DETAILED DESCRIPTION OF THE INVENTION

Before the present system and method of the invention are described, it is to be understood that this invention is not limited to particular systems and methods or combinations described, since such systems and methods and combinations may, of course, vary. It is also to be understood that the terminology used herein is not intended to be limiting, since the scope of the present invention will be limited only by the appended claims.

As used herein, the singular forms "a", "an", and "the" include both singular and plural referents unless the context clearly dictates otherwise.

The terms "comprising", "comprises" and "comprised of" as used herein are synonymous with "including", "includes" or "containing", "contains", and are inclusive or open-ended and do not exclude additional, non-recited members, elements or method steps. It will be appreciated that the terms "comprising", "comprises" and "comprised of" as used herein comprise the terms "consisting of', "consists" and "consists of', as well as the terms "consisting essentially of", "consists essentially" and "consists essentially of".

The recitation of numerical ranges by endpoints includes all numbers and fractions subsumed within the respective ranges, as well as the recited endpoints.

The term "about" or "approximately" as used herein when referring to a measurable value such as a parameter, an amount, a temporal duration, and the like, is meant to encompass variations of +/-20% or less, preferably +/-10% or less, more preferably +/-5% or less, and still more preferably +/-1% or less of and from the specified value, insofar such variations are appropriate to perform in the disclosed invention. It is to be understood that the value to which the modifier "about" or "approximately" refers is itself also specifically, and preferably, disclosed.

Whereas the terms "one or more" or "at least one", such as one or more or at least one member(s) of a group of members, is clear per se, by means of further exemplification, the term encompasses inter alia a reference to any one of said members, or to any two or more of said members, such as, e.g., any ≥3, ≥4, ≥5, ≥6 or ≥7 etc. of said members, and up to all said members.

All references cited in the present specification are hereby incorporated by reference in their entirety. In particular, the teachings of all references herein specifically referred to are incorporated by reference.

Unless otherwise defined, all terms used in disclosing the invention, including technical and scientific terms, have the meaning as commonly understood by one of ordinary skill in the art to which this invention belongs. By means of further guidance, term definitions are included to better appreciate the teaching of the present invention.

Standard reference works setting forth the general principles of recombinant DNA technology include Molecular Cloning: A Laboratory Manual, 4th ed., (Green and Sambrook et al., 2012, Cold Spring Harbor Laboratory Press); Current Protocols in Molecular Biology, ed. Ausubel et al., Greene Publishing and Wiley-Interscience, New York, 1992 (with periodic updates) ("Ausubel et al. 1992"); the series Methods in Enzymology (Academic Press, Inc.); Innis et al., PCR Protocols: A Guide to Methods and Applications, Academic Press: San Diego, 1990; PCR 2: A Practical Approach (M.J. MacPherson, B.D. Hames and G.R. Taylor eds. (1995); Harlow and Lane, eds. (1988) Antibodies, a Laboratory Manual; and Animal Cell Culture (R.I. Freshney, ed. (1987). General principles of microbiology are set forth, for example, in Davis, B. D. et al., Microbiology, 3rd edition, Harper & Row, publishers, Philadelphia, Pa. (1980).

In the following passages, different aspects of the invention are defined in more detail. Each aspect so defined may be combined with any other aspect or aspects unless clearly indicated to the contrary. In particular, any feature indicated as being preferred or advantageous may be combined with any other feature or features indicated as being preferred or advantageous.

Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to a person skilled in the art from this disclosure, in one or more embodiments. Furthermore, while some embodiments described herein include some but not other features included in other embodiments, combinations of features of different embodiments are meant to be within the scope of the invention, and form different embodiments, as would be understood by those in the art. For example, in the appended claims, any of the claimed embodiments can be used in any combination.

In the following detailed description of the invention, reference is made to the accompanying drawings that form a part hereof, and in which are shown by way of illustration only of specific embodiments in which the invention may be practiced. It is to be understood that other embodiments may be utilized and structural or logical changes may be made without departing from the scope of the present invention. The following detailed description, therefore, is not to be taken in a limiting sense, and the scope of the present invention is defined by the appended claims.

Preferred statements (features) and embodiments of this invention are set herein below. Each statements and embodiments of the invention so defined may be combined with any other statement and/or embodiments unless clearly indicated to the contrary. In particular, any feature indicated as being preferred or advantageous may be combined with any other feature or features or statements indicated as being preferred or advantageous.

In an aspect, the invention relates to a method for introducing one or more mutation/genetic marker (preferably linked with a genomic region of interest) in a plant, plant part, or plant population, comprising subjecting a plant or plant part (population) to random mutagenesis and selecting a plant, plant part, population, or progeny thereof comprising a mutation/genetic marker (in particular as introduced by random mutagenesis and/or linked with a genomic region of interest).

In an embodiment, the invention relates to a method for introducing one or more mutation/genetic marker linked with a genomic region of interest in a plant, plant part, or plant population, comprising subjecting a plant or plant part (population) to random mutagenesis and selecting a plant, plant part, population, or progeny thereof comprising a mutation/genetic marker introduced by random mutagenesis and linked with a genomic region of interest.

In a related aspect, the invention relates to the use of random mutagenesis for introducing one or more mutation/genetic marker (in particular as introduced by random mutagenesis) linked with a genomic region of interest in a plant or plant part (population). In an embodiment, the invention relates to the use of random mutagenesis for introducing one or more mutation/genetic marker introduced by random mutagenesis linked with a genomic region of interest in a plant or plant part (population).

In an aspect, the invention relates to a method for generating a plant, plant part, or plant population (in particular comprising a genomic region of interest), comprising subjecting a plant or plant part (population) to random mutagenesis and selecting a plant, plant part, population, or progeny thereof comprising a mutation/genetic marker (in particular as introduced by random mutagenesis and/or linked with a genomic region of interest). In an embodiment, the invention relates to a method for generating a plant, plant part, or plant population comprising a genomic region of interest, comprising subjecting a plant or plant part (population) to random mutagenesis and selecting a plant, plant part, population, or progeny thereof comprising a mutation/genetic marker introduced by random mutagenesis and linked with a genomic region of interest. In a related aspect, the invention relates to the use of random mutagenesis for generating a plant or plant part (population) comprising one or more mutation/genetic marker (in particular as introduced by random mutagenesis) linked with a genomic region of interest. In an embodiment, the invention relates to the use of random mutagenesis for generating a plant or plant part (population) comprising one or more mutation/genetic marker introduced by random mutagenesis and linked with a genomic region of interest.

The genetic mutation/marker may be introduced in a plant or plant population or may be introduced in a plant part or plant part population, such as a cell, tissue or organ, for instance a protoplast or seed or pollen. The skilled person will understand that if a plant or plant part (population) is mutagenized, offspring may need to be generated (and selected for the presence of the mutation) in order ensure that the mutation is present in the genome of the entire plant. In certain preferred embodiments, plant seeds or pollen are subjected to random mutagenesis, preferably chemical mutagenesis, such as EMS induced mutagenesis.

The skilled person will understand that the methods and uses described herein for generating plants may include regenerating plants from mutagenized plant parts, such as mutagenized seeds or mutagenized pollen.

In an aspect, the invention relates to a method for identifying one or more mutation/genetic marker (preferably linked with a genomic region of interest) in a plant or plant part (population), comprising identifying and/or screening for the presence of one or more mutation/genetic marker introduced in a plant or plant part (population) by random mutagenesis (and in particular linked with a genomic region of interest). In an embodiment, the invention relates to method for identifying one or more mutation/genetic marker linked with a genomic region of interest in a plant or plant part (population), comprising identifying and/or screening for the presence of one or more mutation/genetic marker introduced in a plant or plant part (population) by random mutagenesis and linked with a genomic region of interest. In an embodiment, the method further comprises subjecting a plant or plant part (population) to random mutagenesis. In an embodiment, a plant, plant part, or plant population comprising one or more mutation/genetic marker (preferably as generated or introduced by random mutagenesis and/or linked with a genomic region of interest) is selected. In a related aspect, the invention relates to the use of a randomly mutated plant or plant part (population), or progeny thereof, for identifying one or more mutation/genetic marker (in particular as introduced by random mutagenesis) linked with a genomic region of interest. In an embodiment, the invention relates to the use of a randomly mutated plant or plant part (population), or progeny thereof, for identifying one or more mutation/genetic marker introduced by random mutagenesis and linked with a genomic region of interest.

In an aspect, the invention relates to a method for identifying a plant, plant part, or plant population (in particular comprising a genomic region of interest), comprising identifying and/or screening for the presence of one or more mutation/genetic marker introduced in a plant or plant part (population) by random mutagenesis (and in particular linked with a genomic region of interest). In an embodiment, the invention relates to a method for identifying a plant, plant part, or plant population comprising a genomic region of interest, comprising identifying and/or screening for the presence of one or more mutation/genetic marker introduced in a plant or plant part (population) by random mutagenesis and linked with a genomic region of interest. In an embodiment, the method further comprises subjecting a plant or plant part (population) to random mutagenesis. In an embodiment, a plant, plant part, or plant population comprising one or more mutation/genetic marker (preferably as generated or introduced by random mutagenesis and/or linked with a genomic region of interest) is selected. In a related aspect, the invention relates to the use of a randomly mutated plant or plant part (population), or progeny thereof, for identifying a plant or plant part comprising one or more mutation/genetic marker (in particular as introduced by random mutagenesis) linked with a genomic region of interest. In an embodiment, the invention relates to the use of a randomly mutated plant or plant part (population), or progeny thereof, for identifying a plant or plant part comprising one or more mutation/genetic marker introduced by random mutagenesis and linked with a genomic region of interest.

In an aspect, the invention relates to a method for fine mapping a genomic region of interest in a plant or plant part, comprising identifying and/or screening for the presence of one or more mutation/genetic marker introduced in a plant or plant part (population) by random mutagenesis (and in particular linked with a genomic region of interest). In an embodiment, the invention relates to a method for fine mapping a genomic region of interest in a plant or plant part, comprising identifying and/or screening for the presence of one or more mutation/genetic marker introduced in a plant or plant part (population) by random mutagenesis and linked with a genomic region of interest. In an embodiment, a plant, plant part, or plant population comprising one or more mutation/genetic marker (preferably as generated or introduced by random mutagenesis and/or linked with a genomic region of interest) is selected. In a related aspect, the invention relates to the use of random mutagenesis for fine mapping a genomic region of interest in a plant or plant part (population).

The term "plant" according to the present invention includes whole plants or parts of such a whole plant. Whole plants preferably are seed plants, or a crop. "Parts of a plant" are e.g. shoot vegetative organs/structures, e.g., leaves, stems and tubers; roots, flowers and floral organs/structures, e.g. bracts, sepals, petals, stamens, carpels, anthers and ovules; pollen, seed, including embryo, endosperm, and seed coat; fruit and the mature ovary; plant tissue, e.g. vascular tissue, ground tissue, and the like; and cells, e.g. guard cells, egg cells, pollen, trichomes and the like; and progeny of the same. Parts of plants may be attached to or separate from a whole intact plant. Such parts of a plant include, but are not limited to, organs, tissues, and cells of a plant, and preferably pollen (or seeds). A "plant cell" is a structural and physiological unit of a plant, comprising a protoplast and a cell wall. The plant cell may be in form of an isolated single cell or a cultured cell, or as a part of higher organized unit such as, for example, plant tissue, a plant organ, or a whole plant. "Plant cell culture" means cultures of plant units such as, for example, protoplasts, cell culture cells, cells in plant tissues, pollen, pollen tubes, ovules, embryo sacs, zygotes and embryos at various stages of development. "Plant material" refers to leaves, stems, roots, flowers or flower parts, fruits, pollen, egg cells, zygotes, pollen, seeds, cuttings, cell or tissue cultures, or any other part or product of a plant. This also includes callus or callus tissue as well as extracts (such as extracts from taproots) or samples. A "plant organ" is a distinct and visibly structured and differentiated part of a plant such as a root, stem, leaf, flower bud, or embryo. "Plant tissue" as used herein means a group of plant cells organized into a structural and functional unit. Any tissue of a plant in planta or in culture is included. This term includes, but is not limited to, whole plants, plant organs, plant pollen, plant seeds, tissue culture and any groups of plant cells organized into structural and/or functional units. The use of this term in conjunction with, or in the absence of, any specific type of plant tissue as listed above or otherwise embraced by this definition is not intended to be exclusive of any other type of plant tissue. In certain embodiments, the plant part or derivative is not (functional) propagation material, such as germplasm, a seed, or plant embryo or other material from which a plant can be regenerated. In certain embodiments, the plant part or derivative does not comprise (functional) male and female reproductive organs. In certain embodiments, the plant part or derivative is or comprises propagation material, but propagation material which does not or cannot be used (anymore) to produce or generate new plants, such as propagation material which have been chemically, mechanically or otherwise rendered non-functional, for instance by heat treatment, acid treatment, compaction, crushing, chopping, etc. In certain embodiments, the plant part or derivative is (functional) propagation material, such as germplasm, a seed, or plant embryo or other material from which a plant can be regenerated. In certain embodiments, the plant part or derivative comprises (functional) male and female reproductive organs.

As used herein unless clearly indicated otherwise, the term "plant" intended to mean a plant at any developmental stage.

As used herein, the term "plant (part) population" may be used interchangeably with population of plants or plant parts. A plant (part) population preferably comprises a multitude of individual plants (or plant parts thereof), such as preferably at least 10, such as 20, 30, 40, 50, 60, 70, 80, or 90, more preferably at least 100, such as 200, 300, 400, 500, 600, 700, 800, or 900, even more preferably at least 1000, such as at least 10000 or at least 100000.

In certain embodiments, the plant population (or plant parts thereof) is a plant line, strain, or variety. In certain embodiments, the plant population (or plant parts thereof) is not a plant line, strain, or variety. In certain embodiments, the plant population (or plant parts thereof) is an inbred plant line, strain, or variety. In certain embodiments, the plant population (or plant parts thereof) is not an inbred plant line, strain, or variety. In certain embodiments, the plant population (or plant parts thereof) is an outbred plant line, strain, or variety. In certain embodiments, the plant population (or plant parts thereof) is not an outbred plant line, strain, or variety.

In certain embodiments, the plants, plant parts, or plant population have a (average) degree of homozygosity of at least 50%, preferably at least 60%, more preferably at least 70%, most preferably at least 80%, such as at least 90%. In certain embodiments, the plants, plant parts, or plant population have a (average) degree of homozygosity of at least 50%, preferably at least 60%, more preferably at least 70%, most preferably at least 80%, such as at least 90% within 20 cM of a genomic region of interest.

As used herein, a "genomic region of interest" can be any genomic region (i.e. corresponding to a particular a genomic nucleotide sequence) within the genome of a plant, such as a single gene, but also larger genomic segments comprising one or more genes, such as a quantitive trait locus (QTL). The gene may or may not encode a protein. The gene may or may not encode an untranslated RNA, such as rRNA or miRNA. As used herein, the term genomic region of interest can be used interchangeably with (genomic) locus of interest or (genomic) trait of interest, or can be said to confer a trait of interest. Preferably, the genomic region of interest is associated with an agronomically relevant or important phenotype. An agronomically important phenotype in the context of the present invention is a phenotype of a plant, which exhibits one or more novel or optimized trait(s) that provide an improved agricultural performance with respect to e.g. yield, biomass, architecture, morphology, fertility, pollen shedding, nutrient partitioning, photosynthesis, carbon sequestration, disease resistance, abiotic and biotic stress tolerance, herbicide tolerance, hormone signaling, and other trait categories. An agronomically important phenotype may be caused by any one or a combination of one or more allelic variations in one or more coding, noncoding or regulatory regions of the genetic material of the plant. An agronomically important phenotype may exhibit one or more polygenic traits.

The term "sequence" when used herein relates to nucleotide sequence(s), polynucleotide(s), nucleic acid sequence(s), nucleic acid(s), nucleic acid molecule, peptides, polypeptides and proteins, depending on the context in which the term "sequence" is used.

The terms "nucleotide sequence(s)", "polynucleotide(s)", "nucleic acid sequence(s)", "nucleic acid(s)", "nucleic acid molecule" are used interchangeably herein and refer to nucleotides, either ribonucleotides or deoxyribonucleotides or a combination of both, in a polymeric unbranched form of any length. Nucleic acid sequences include DNA, cDNA, genomic DNA, RNA, synthetic forms and mixed polymers, both sense and antisense strands, or may contain non-natural or derivatized nucleotide bases, as will be readily appreciated by those skilled in the art.

When used herein, the term "polypeptide" or "protein" (both terms are used interchangeably herein) means a peptide, a protein, or a polypeptide which encompasses amino acid chains of a given length, wherein the amino acid residues are linked by covalent peptide bonds. However, peptidomimetics of such proteins/polypeptides wherein amino acid(s) and/or peptide bond(s) have been replaced by functional analogs are also encompassed by the invention as well as other than the 20 gene-encoded amino acids, such as selenocysteine. Peptides, oligopeptides and proteins may be termed polypeptides. The term polypeptide also refers to, and does not exclude, modifications of the polypeptide, e.g., glycosylation, acetylation, phosphorylation and the like. Such modifications are well described in basic texts and in more detailed monographs, as well as in the research literature.

The term "gene" when used herein refers to a polymeric form of nucleotides of any length, either ribonucleotides or desoxyribonucleotides. The term includes double- and single-stranded DNA and RNA. It also includes known types of modifications, for example, methylation, "caps", substitutions of one or more of the naturally occurring nucleotides with an analog. Preferably, a gene comprises a coding sequence encoding the herein defined polypeptide. A "coding sequence" is a nucleotide sequence which is transcribed into mRNA and/or translated into a polypeptide when placed or being under the control of appropriate regulatory sequences. The boundaries of the coding sequence are determined by a translation start codon at the 5'-terminus and a translation stop codon at the 3'-terminus. A coding sequence can include, but is not limited to mRNA, cDNA, recombinant nucleic acid sequences or genomic DNA, while introns may be present as well under certain circumstances.

In certain embodiments, the genomic region of interest as described herein is in a (genomic) region/area or lies in a (genomic) region/area of low polymorphism. In certain embodiments, the genomic region of interest as described herein is in a local (genomic) region/area or lies in a local (genomic) region/area of local low polymorphism. This means that in the vicinity of the genomic region of interest no or a limited (average) number of polymorphisms can be found (for instance in a population), or that the (average) number of polymorphisms found in the vicinity of the genomic region of interest is lower that the (average) number of polymorphisms found elsewhere (for instance elsewhere on the same chromosome or elsewhere in the entire genome). This may also mean that in the vicinity of the genomic region of interest no or a low (average) density of polymorphisms is present (for instance in a population), or that the (average) density of polymorphisms found in the vicinity of the genomic region of interest is lower that the (average) density of polymorphisms found elsewhere (for instance elsewhere on the same chromosome or elsewhere in the entire genome). This may also mean that in the vicinity of the genomic region of interest a low (average) degree of polymorphism is observed (for instance in a population), or that the (average) degree of polymorphism found in the vicinity of the genomic region of interest is lower that the (average) degree of polymorphism found elsewhere (for instance elsewhere on the same chromosome or elsewhere in the entire genome). Accordingly, introducing the mutation/marker as described herein increases the (average) number of polymorphisms, the (average) polymorphism density, and/or the (average) degree of polymorphism. The skilled person will understand that when referring to a region/area of low polymorphisms, reference is made to the wild-type situation.

In certain embodiments, less than 10 (unique) polymorphisms (for instance SNPs), such as preferably less than 5 (unique) polymorphisms, more preferably less than 2 (unique) polymorphisms are present (or can be identified) in a plant or plant part (population) within 100 kb, preferably within 1 Mb upstream and/or downstream of a genomic region of interest, such as a genomic region of interest in an area/region of low polymorphism. In certain embodiments, less than 10 (unique) polymorphisms (for instance SNPs), such as preferably less than 5 (unique) polymorphisms, more preferably less than 2 (unique) polymorphisms are present (or can be identified) in a plant or plant part (population) within 100 kb, preferably within 1 Mb upstream of a genomic region of interest, such as a genomic region of interest in an area/region of low polymorphism. In certain embodiments, less than 10 (unique) polymorphisms (for instance SNPs), such as preferably less than 5 (unique) polymorphisms, more preferably less than 2 (unique) polymorphisms are present (or can be identified) in a plant or plant part (population) within 100 kb, preferably within 1 Mb downstream of a genomic region of interest, such as a genomic region of interest in an area/region of low polymorphism. In certain embodiments, less than 10 (unique) polymorphisms (for instance SNPs), such as preferably less than 5 (unique) polymorphisms, more preferably less than 2 (unique) polymorphisms are present (or can be identified) in a plant or plant part (population) within 100 kb, preferably within 1 Mb upstream and downstream (separately or combined) of a genomic region of interest, such as a genomic region of interest in an area/region of low polymorphism. It will be understood that "unique" polymorphisms preferably are those that allow specific identification of the genomic region (allele) of interest. In certain embodiments, polymorphisms which do not allow specific identification of the genomic region of interest (allele) may be disregarded.

In certain embodiments, less than 10 (unique) polymorphisms (for instance SNPs), such as preferably less than 5 (unique) polymorphisms, more preferably less than 2 (unique) polymorphisms are present (or can be identified) in a plant or plant part (population) within 1 cM, such as within 2 cM, preferably within 5 cM, more preferably within 10 cM, most preferably within 20 cM upstream and/or downstream of a genomic region of interest, such as a genomic region of interest in an area/region of low polymorphism. In certain embodiments, less than 10 (unique) polymorphisms (for instance SNPs), such as preferably less than 5 (unique) polymorphisms, more preferably less than 2 (unique) polymorphisms are present (or can be identified) in a plant or plant part (population) within 1 cM, such as within 2 cM, preferably within 5 cM, more preferably within 10 cM, most preferably within 20 cM upstream of a genomic region of interest, such as a genomic region of interest in an area/region of low polymorphism. In certain embodiments, less than 10 (unique) polymorphisms (for instance SNPs), such as preferably less than 5 (unique) polymorphisms, more preferably less than 2 (unique) polymorphisms are present (or can be identified) in a plant or plant part (population) within 1 cM, such as within 2 cM, preferably within 5 cM, more preferably within 10 cM, most preferably within 20 cM downstream of a genomic region of interest, such as a genomic region of interest in an area/region of low polymorphism. In certain embodiments, less than 10 (unique) polymorphisms (for instance SNPs), such as preferably less than 5 (unique) polymorphisms, more preferably less than 2 (unique) polymorphisms are present (or can be identified) in a plant or plant part (population) within 1 cM, such as within 2 cM, preferably within 5 cM, more preferably within 10 cM, most preferably within 20 cM upstream and downstream (separately or combined) of a genomic region of interest, such as a genomic region of interest in an area/region of low polymorphism. It will be understood that "unique" polymorphisms preferably are those that allow specific identification of the genomic region (allele) of interest. In certain embodiments, polymorphisms which do not allow specific identification of the genomic region of interest (allele) may be disregarded.

In certain embodiments, less than 10 (unique) polymorphisms (for instance SNPs), such as preferably less than 5 (unique) polymorphisms, more preferably less than 2 (unique) polymorphisms upstream and/or downstream of a genomic region of interest, such as a genomic region of interest in an area/region of low polymorphism have an LOD score of at least 3, preferably at least 4. In certain embodiments, less than 10 (unique) polymorphisms (for instance SNPs), such as preferably less than 5 (unique) polymorphisms, more preferably less than 2 (unique) polymorphisms upstream of a genomic region of interest, such as a genomic region of interest in an area/region of low polymorphism have an LOD score of at least 3, preferably at least 4. In certain embodiments, less than 10 (unique) polymorphisms (for instance SNPs), such as preferably less than 5 (unique) polymorphisms, more preferably less than 2 (unique) polymorphisms downstream of a genomic region of interest, such as a genomic region of interest in an area/region of low polymorphism have an LOD score of at least 3, preferably at least 4. In certain embodiments, less than 10 (unique) polymorphisms (for instance SNPs), such as preferably less than 5 (unique) polymorphisms, more preferably less than 2 (unique) polymorphisms upstream and downstream (separately or combined) of a genomic region of interest, such as a genomic region of interest in an area/region of low polymorphism have an LOD score of at least 3, preferably at least 4. It will be understood that "unique" polymorphisms preferably are those that allow specific identification of the genomic region (allele) of interest. In certain embodiments, polymorphisms which do not allow specific identification of the genomic region of interest (allele) may be disregarded.

In certain embodiments, the level of polymorphism or the degree of polymorphism (e.g. θₛ) upstream and/or downstream of a genomic region of interest, such as a genomic region of interest in an area/region of low polymorphism, is at most 10%, preferably at most 5%, more preferably at most 2%, most preferably at most 1%, preferably within a region of 1 Mb, such as preferably 5 Mb or 10 Mb upstream and/or downstream of the genomic region of interest. In certain embodiments, the level of polymorphism or the degree of polymorphism (e.g. θₛ) upstream of a genomic region of interest, such as a genomic region of interest in an area/region of low polymorphism, is at most 10%, preferably at most 5%, more preferably at most 2%, most preferably at most 1%, preferably within a region of 1 Mb, such as preferably 5 Mb or 10 Mb upstream of the genomic region of interest. In certain embodiments, the level of polymorphism or the degree of polymorphism (e.g. θₛ) downstream of a genomic region of interest, such as a genomic region of interest in an area/region of low polymorphism, is at most 10%, preferably at most 5%, more preferably at most 2%, most preferably at most 1%, preferably within a region of 1 Mb, such as preferably 5 Mb or 10 Mb downstream of the genomic region of interest. In certain embodiments, the level of polymorphism or the degree of polymorphism (e.g. θₛ) upstream and downstream of a genomic region of interest, such as a genomic region of interest in an area/region of low polymorphism, is at most 10%, preferably at most 5%, more preferably at most 2%, most preferably at most 1%, preferably within a region of 1 Mb, such as preferably 5 Mb or 10 Mb upstream and downstream of the genomic region of interest. The skilled person will appreciate that additional means to determine the level or degree of polymorphism can be applied.

In certain embodiments, the mutations/markers as described herein are flanking a genomic region of interest, such as a gene, QTL or other locus conferring a trait to a plant. The skilled person will understand that a mutation/marker flanking the genomic region of interest is located suitably close to the genomic region of interest in order for the mutation/marker to be considered "linked" with the genomic region of interest, as described herein elsewhere. In certain embodiments, one or more mutation/marker is flanking the 5' end on the genomic region of interest. In certain embodiments, one or more mutation/marker is flanking the 3' end on the genomic region of interest.

In certain embodiments, the mutations/markers as described herein are more than one mutation/marker, such as in certain embodiments two mutations/markers. In certain embodiments, one or more mutation/marker is flanking the genomic region of interest and one or more mutation/marker is located within the genomic region of interest. In certain embodiments, more than one mutation/marker is located within the genomic region of interest. In certain embodiments, more than one mutation/marker is flanking the genomic region of interest. In certain embodiments, two mutations/markers are flanking the genomic region of interest. In certain embodiments, one mutation/marker is flanking the 5' end on the genomic region of interest. In certain embodiments, one mutation/marker is flanking the 3' end on the genomic region of interest. In certain embodiments, one (or more) mutation/marker is flanking the 5' end on the genomic region of interest and one (or more) mutation/marker is flanking the 3' end on the genomic region of interest.

In certain embodiments, one or more mutation/marker as described herein is located within 20 cM (centimorgan), such as preferably 10 cM, more preferably 2 cM, even more preferably 1 cM, most preferably 0.25 cM from a genomic region of interest. In certain embodiments, one or more mutation/marker as described herein is located within 20 cM, such as preferably 10 cM, more preferably 2 cM, even more preferably 1 cM, most preferably 0.25 cM from the 5' end of a genomic region of interest. In certain embodiments, one or more mutation/marker as described herein is located within 20 cM, such as preferably 10 cM, more preferably 2 cM, even more preferably 1 cM, most preferably 0.25 cM from the 3' end of a genomic region of interest. In certain embodiments, one or more mutation/marker as described herein is located within 20 cM, such as preferably 10 cM, more preferably 2 cM, even more preferably 1 cM, most preferably 0.25 cM from the 5' end or the 3' end of a genomic region of interest. In certain embodiments, one or more mutation/marker as described herein is located within 20 cM, such as preferably 10 cM, more preferably 2 cM, even more preferably 1 cM, most preferably 0.25 cM from the 5' end of a genomic region of interest an one or more mutation/marker as described herein is located within 20 cM, such as preferably 10 cM, more preferably 2 cM, even more preferably 1 cM, most preferably 0.25 cM from the 5' end of a genomic region of interest.

In certain embodiments, the LOD (logarithm of odds) score or one or more mutation/marker as described herein and the genomic region of interest is at least 3, such as preferably at least 3.5, more preferably at least 4, even more preferably at least 4.5, most preferably at least 5. In certain embodiments, the LOD score or one or more 5' mutation/marker as described herein and the genomic region of interest is at least 3, such as preferably at least 3.5, more preferably at least 4, even more preferably at least 4.5, most preferably at least 5. In certain embodiments, the LOD score or one or more 3' mutation/marker as described herein and the genomic region of interest is at least 3, such as preferably at least 3.5, more preferably at least 4, even more preferably at least 4.5, most preferably at least 5. In certain embodiments, the LOD score or one or more 5' or 3' mutation/marker as described herein and the genomic region of interest is at least 3, such as preferably at least 3.5, more preferably at least 4, even more preferably at least 4.5, most preferably at least 5. In certain embodiments, the LOD score or one or more 5' mutation/marker as described herein and the genomic region of interest is at least 3, such as preferably at least 3.5, more preferably at least 4, even more preferably at least 4.5, most preferably at least 5 and the LOD score or one or more 3' mutation/marker as described herein and the genomic region of interest is at least 3, such as preferably at least 3.5, more preferably at least 4, even more preferably at least 4.5, most preferably at least 5.

In certain embodiments, one or more mutation/marker as described herein is located within 20 Mb (megabase), such as preferably 10 Mb, more preferably 5 Mb, even more preferably 2 Mb, most preferably 1 Mb from a genomic region of interest. In certain embodiments, one or more mutation/marker as described herein is located within 20 Mb, such as preferably 10 Mb, more preferably 5 Mb, even more preferably 2 Mb, most preferably 1 Mb from the 5' end of a genomic region of interest. In certain embodiments, one or more mutation/marker as described herein is located within 20 Mb, such as preferably 10 Mb, more preferably 5 Mb, even more preferably 2 Mb, most preferably 1 Mb from the 3' end of a genomic region of interest. In certain embodiments, one or more mutation/marker as described herein is located within 20 Mb, such as preferably 10 Mb, more preferably 5 Mb, even more preferably 2 Mb, most preferably 1 Mb from the 5' end or the 3' end of a genomic region of interest. In certain embodiments, one or more mutation/marker as described herein is located within 20 Mb, such as preferably 10 Mb, more preferably 5 Mb, even more preferably 2 Mb, most preferably 1 Mb from the 5' end of a genomic region of interest an one or more mutation/marker as described herein is located within 20 Mb, such as preferably 10 Mb, more preferably 5 Mb, even more preferably 2 Mb, most preferably 1 Mb from the 5' end of a genomic region of interest.

In certain embodiments, the mutations/markers as described herein are located within a genomic region of interest, such as a gene, QTL or other locus conferring a trait to a plant. For instance, a QTL may comprise several genes and a mutation/marker may be located between genes or even within one gene. If a mutation/marker is located within a gene, preferably, as described herein elsewhere, the mutation/marker is not a functional marker (i.e. the marker in itself does not cause a phenotypic effect). For instance, the mutation/maker may be in an intron of a gene (not affecting splicing). If the mutation/marker is within the coding sequence of a gene, preferably the mutation does alter the protein sequence (for instance as a consequence of codon degeneracy). Preferably, if the mutation results in an altered protein sequence, the mutation does not cause a phenotypic effect. It will be understood that markers located within a genomic region of interest in certain embodiments are particularly advantageous are these are most closely linked to the genomic region of interest.

A used herein, the term "endogenous" refers to a gene or (marker) allele which is present in its natural genomic location. The term "endogenous" can be used interchangeably with "native". This does not however exclude the presence of one or more nucleic acid differences with the wild-type allele, due to naturally occurring polymorphisms. In particular embodiments, the difference with a wild-type allele can be limited to less than 9 preferably less than 6, more particularly less than 3 nucleotide differences. More particularly, the difference with the wildtype sequence can be in only one nucleotide. A used herein, the term "endogenous" may refer to a marker/gene or allele which has not been introduced into the plant (or its ancestry) by genetic engineering techniques or (artificial) mutagenesis. The term "endogenous" can be used interchangeously with "native" or "wild type". Naturally occurring polymorphisms can all be considered endogenous, native, and/or wild type, in contrast to artificially introduced mutations or polymorphisms. Non-naturally occurring polymorphisms or mutations, such as those introduced by random mutagenesis, may be considered exogenous, non-native, or genetically engineered.

In certain preferred embodiments, the mutations or genetic markers of the present invention are artificial, i.e. non-natural or exogenous (non-endogenous). In certain preferred embodiments, the mutations or genetic markers of the present invention are unique in a plant or within a population of plants or plant parts. In certain preferred embodiments, the mutations or genetic markers of the present invention are artificial. In certain preferred embodiments, the mutations or genetic markers of the present invention are not naturally present in a plant or within a population of plants or plant parts, or in any case at most in 1%, preferably at most in 0.1% in a plant or within a population of plants or plant parts. Accordingly, in certain embodiments, the mutation or marker is not endogenous. The skilled person will appreciate that whether or not a nutation/marker is naturally occurring may or may not depend on the plant population size. For instance, a mutation/marker may be unique for a particular sub-population but not necessary unique for the entire population. For instance, a mutation/marker may be unique for some particular plant line or strain, or for a particular group of plant lines or strains.

In certain embodiments, a single mutation/marker may not be unique in a plant (part) or plant (part) population, but a combination of mutations/markers, such as two, three, four, five, or more mutations/markers, such as in particular two mutations/markers (for instance a mutation/marker flanking the 5' end of a genomic region of interest and a mutation/marker flanking the 3' end of a genomic region of interest), is unique in a plant (part) or plant (part) population (or in any case at most in 1%, preferably at most in 0.1% in a plant or within a population of plants or plant parts). Accordingly, in such cases, whereas each of the single mutations/markers may have a certain prevalence in a plant (population) or plant part (population), the specific combination of mutations/markers is unique. Accordingly, a combination of mutations/marker according to the present invention may be linked to a genomic region of interest and may allow identification of the genomic region of interest or allow identification of a plant or plant part (population), such as a plant or plant part (population) comprising a genomic region of interest such as a relevant trait.

In certain embodiments, the mutation/marker as described herein does not cause a phenotypic effect. As such, the mutation/marker is preferably not a functional mutation. Whereas the mutation/marker may be linked with a genomic region of interest, which genomic region of interest may cause a phenotypic effect, the marker itself does not. In certain embodiments, the mutation/marker does not alter RNA and/or protein expression of a gene, RNA and/or protein stability, and/or RNA and/or protein activity. In certain embodiments, the mutation/marker does not alter RNA and/or protein function. Accordingly, while the marker in itself does not have a functional effect, it may nevertheless be selectable as being linked with a genomic region of interest, such as a genomic region of interest (or locus) encoding a trait.

The term "locus" (loci plural) means a specific place or places or a site on a chromosome where a genomic region of interest, for example a QTL, a gene or genetic marker, is found. As used herein, the term "quantitative trait locus" or "QTL" has its ordinary meaning known in the art. By means of further guidance, and without limitation, a QTL may refer to a region of DNA that is associated with the differential expression of a quantitative phenotypic trait in at least one genetic background, e.g., in at least one breeding population. The region of the QTL encompasses or is closely linked to the gene or genes that affect the trait in question. An "allele of a QTL" can comprise multiple genes or other genetic factors within a contiguous genomic region or linkage group, such as a haplotype. An allele of a QTL can denote a haplotype within a specified window wherein said window is a contiguous genomic region that can be defined, and tracked, with a set of one or more polymorphic markers. A haplotype can be defined by the unique fingerprint of alleles at each marker within the specified window. A QTL may encode for one or more alleles that affect the expressivity of a continuously distributed (quantitative) phenotype. In certain embodiments, the QTL as described herein may be homozygous. In certain embodiments, the QTL as described herein may be heterozygous.

As used herein, the term "allele" or "alleles" refers to one or more alternative forms, i.e. different nucleotide sequences, of a locus.

The allelic variation as used herein may be naturally occurring (endogenous, native, or wild type allele) or non-naturally occurring (mutant allele). A naturally occurring allelic variation is to be understood to be present at a certain frequency in a given population. In contrast, a non-naturally occurring allelic variation is not present in a given population. In a preferred embodiment, the allelic variation is non-naturally occurring or preferably occurring in less than 1%, such as preferably less than 0.1% of a given population. It will be understood that all these types of mutations described above are encompassed by the term allelic variation as used herein.

As used herein, the term "mutant alleles" or "mutation" of alleles include alleles having one or more mutations, such as insertions, deletions, stop codons, base changes (e.g. , transitions or transversions), or alterations in splice junctions, which may or may not give rise to altered gene products. Modifications in alleles may arise in coding or noncoding regions (e.g. promoter regions, exons, introns or splice junctions).

The markers or marker alleles as described herein may be diagnostic marker alleles which are useable for identifying plants or plant parts having a particular genomic region of interest (allele).

As used herein, the terms "introgression", "introgressed" and "introgressing" refer to both a natural and artificial process whereby chromosomal fragments or genes of one species, variety or cultivar are moved into the genome of another species, variety or cultivar, by crossing those species. The process may optionally be completed by backcrossing to the recurrent parent. For example, introgression of a desired allele at a specified locus can be transmitted to at least one progeny via a sexual cross between two parents of the same species, where at least one of the parents has the desired allele in its genome. Alternatively, for example, transmission of an allele can occur by recombination between two donor genomes, e.g., in a fused protoplast, where at least one of the donor protoplasts has the desired allele in its genome. The desired allele can be, e.g., detected by a marker that is associated with a phenotype, at a QTL, a transgene, or the like. In any case, offspring comprising the desired allele can be repeatedly backcrossed to a line having a desired genetic background and selected for the desired allele, to result in the allele becoming fixed in a selected genetic background. The process of "introgressing" is often referred to as "backcrossing" when the process is repeated two or more times. "Introgression fragment" or "introgression segment" or "introgression region" refers to a chromosome fragment (or chromosome part or region) which has been introduced into another plant of the same or related species either artificially or naturally such as by crossing or traditional breeding techniques, such as backcrossing, i.e. the introgressed fragment is the result of breeding methods referred to by the verb "to introgress" (such as backcrossing). It is understood that the term "introgression fragment" never includes a whole chromosome, but only a part of a chromosome. The introgression fragment can be large, e.g. even three quarter or half of a chromosome, but is preferably smaller, such as about 15 Mb or less, such as about 10 Mb or less, about 9 Mb or less, about 8 Mb or less, about 7 Mb or less, about 6 Mb or less, about 5 Mb or less, about 4 Mb or less, about 3 Mb or less, about 2.5 Mb or 2 Mb or less, about 1 Mb (equals 1,000,000 base pairs) or less, or about 0.5 Mb (equals 500,000 base pairs) or less, such as about 200,000 bp (equals 200 kilo base pairs) or less, about 100,000 bp (100 kb) or less, about 50,000 bp (50 kb) or less, about 25,000 bp (25 kb) or less.

A genetic element, an introgression fragment, or a gene or allele conferring a trait or a marker as described herein is said to be "obtainable from" or can be "obtained from" or "derivable from" or can be "derived from" or "as present in" or "as found in" a plant or plant part as described herein elsewhere if it can be transferred from the plant in which it is present into another plant in which it is not present (such as a line or variety) using traditional breeding techniques without resulting in a phenotypic change of the recipient plant apart from the addition of the trait conferred by the genetic element, locus, introgression fragment, gene or allele or a marker as described herein. The terms are used interchangeably and the genetic element, locus, introgression fragment, gene, marker or allele can thus be transferred into any other genetic background lacking the trait. Not only pants comprising the genetic element, locus, introgression fragment, gene, marker, or allele can be used, but also progeny/descendants from such plants which have been selected to retain the genetic element, locus, introgression fragment, gene, marker, or allele, can be used and are encompassed herein. Whether a plant (or genomic DNA, cell or tissue of a plant) comprises the same genetic element, locus, introgression fragment, gene, marker, or allele as obtainable from such plant can be determined by the skilled person using one or more techniques known in the art, such as phenotypic assays, whole genome sequencing, molecular marker analysis, trait mapping, chromosome painting, allelism tests and the like, or combinations of techniques. It will be understood that transgenic plants may also be encompassed.

As used herein the terms "genetic engineering", "transformation" and "genetic modification" are all used herein as synonyms for the transfer of isolated and cloned genes into the DNA, usually the chromosomal DNA or genome, of another organism.

"Transgenic" or "genetically modified organisms" (GMOs) as used herein are organisms whose genetic material has been altered using techniques generally known as "recombinant DNA technology". Recombinant DNA technology encompasses the ability to combine DNA molecules from different sources into one molecule ex vivo (e.g. in a test tube). This terminology generally does not cover organisms whose genetic composition has been altered by conventional cross-breeding or by "mutagenesis" breeding, as these methods predate the discovery of recombinant DNA techniques. "Non-transgenic" as used herein refers to plants and food products derived from plants that are not "transgenic" or "genetically modified organisms" as defined above.

"Transgene" or "chimeric gene" refers to a genetic locus comprising a DNA sequence, such as a recombinant gene, which has been introduced into the genome of a plant by transformation, such as Agrobacterium mediated transformation. A plant comprising a transgene stably integrated into its genome is referred to as "transgenic plant".

According to the invention, the nucleic acid modification is effected by random mutagenesis. Cells or organisms may be exposed to mutagens such as UV, X-ray, or gamma ray radiation or mutagenic chemicals (such as for instance such as ethyl methanesulfonate (EMS), ethylnitrosourea (ENU), or dimethylsulfate (DMS), and mutants with desired characteristics are then selected. Mutants can for instance be identified by TILLING (Targeting Induced Local Lesions in Genomes). The method combines mutagenesis, such as mutagenesis using a chemical mutagen such as ethyl methanesulfonate (EMS) with a sensitive DNA screening-technique that identifies single base mutations/point mutations in a target gene. The TILLING method relies on the formation of DNA heteroduplexes that are formed when multiple alleles are amplified by PCR and are then heated and slowly cooled. A "bubble" forms at the mismatch of the two DNA strands, which is then cleaved by a single stranded nuclease. The products are then separated by size, such as by HPLC. See also McCallum et al. "Targeted screening for induced mutations"; Nat Biotechnol. 2000 Apr;18(4):455-7 and McCallum et al. "Targeting Induced Local Lesions IN Genomes (TILLING) for plant functional genomics"; Plant Physiol. 2000 Jun;123(2):439-42, both incorporated by reference in their entirety. By means of further example, and without limitation, the methodologies described in the following publications, incorporated by reference in their entirety, may be adopted according to the present invention, such as in connection with EMS mutagenesis: Till et al. "Discovery of induced point mutations in maize genes by TILLING"; BMC Plant Biol. 2004 Jul 28;4:12; and Weil & Monde "Getting the point-mutations in maize" Crop Sci 2007; 47 S60-S67. The skilled person will understand that depending on the mutagen dose (irradiation of chemical) the (average) mutation density can be varied or fixed.

In certain embodiments, the random mutagenesis is single nucleotide mutagenesis. In certain embodiments, the random mutagenesis is chemical mutagenesis, preferably EMS mutagenesis.

In certain embodiments, the random mutagenesis results in an average number of mutations ranging from 1 to 100 mutations per 1 Mb. In certain embodiments, the random mutagenesis results in an average number of mutations ranging from 1 to 100 mutations per 100 kb. In certain embodiments, the random mutagenesis results in an average number of mutations ranging from 1 to 50 mutations per 1 Mb. In certain embodiments, the random mutagenesis results in an average number of mutations ranging from 1 to 50 mutations per 100 kb. In certain embodiments, the random mutagenesis results in an average number of mutations ranging from 1 to 20 mutations per 1 Mb. In certain embodiments, the random mutagenesis results in an average number of mutations ranging from 1 to 100 mutations per 20 kb.

In certain embodiments, the random mutagenesis is single nucleotide mutagenesis and results in an average number of mutations ranging from 1 to 100 mutations per 1 Mb. In certain embodiments, the random mutagenesis is single nucleotide mutagenesis and results in an average number of mutations ranging from 1 to 100 mutations per 100 kb. In certain embodiments, the random mutagenesis is single nucleotide mutagenesis and results in an average number of mutations ranging from 1 to 50 mutations per 1 Mb. In certain embodiments, the random mutagenesis is single nucleotide mutagenesis and results in an average number of mutations ranging from 1 to 50 mutations per 100 kb. In certain embodiments, the random mutagenesis is single nucleotide mutagenesis and results in an average number of mutations ranging from 1 to 20 mutations per 1 Mb. In certain embodiments, the random mutagenesis is single nucleotide mutagenesis and results in an average number of mutations ranging from 1 to 100 mutations per 20 kb.

In certain embodiments, the random mutagenesis is chemical mutagenesis, preferably EMS mutagenesis and results in an average number of mutations ranging from 1 to 100 mutations per 1 Mb. In certain embodiments, the random mutagenesis is chemical mutagenesis, preferably EMS mutagenesis and results in an average number of mutations ranging from 1 to 100 mutations per 100 kb. In certain embodiments, the random mutagenesis is chemical mutagenesis, preferably EMS mutagenesis and results in an average number of mutations ranging from 1 to 50 mutations per 1 Mb. In certain embodiments, the random mutagenesis is chemical mutagenesis, preferably EMS mutagenesis and results in an average number of mutations ranging from 1 to 50 mutations per 100 kb. In certain embodiments, the random mutagenesis is chemical mutagenesis, preferably EMS mutagenesis and results in an average number of mutations ranging from 1 to 20 mutations per 1 Mb. In certain embodiments, the random mutagenesis is chemical mutagenesis, preferably EMS mutagenesis and results in an average number of mutations ranging from 1 to 100 mutations per 20 kb.

In certain embodiments, the plant, plant part, or population as described herein comprises one or more mutation(s) or polymorphism(s). In certain embodiments, each member or individual of the population comprises one or more mutation(s) or polymorphism(s). It is to be understood that such mutation(s) or polymorphism(s) may be considered (an) allelic variation(s) as described herein elsewhere. In certain embodiments, said mutation or polymorphism is a point mutation. In certain embodiments, said mutation or polymorphism is an indel. In certain embodiments, said mutation or polymorphism results in a frameshift of a protein coding sequence. In certain embodiments, said mutation or polymorphism is a nonsense mutation. In certain embodiments, said mutation or polymorphism results in a knockout or knockdown of a gene and/or protein. In certain embodiments, said mutation or polymorphism is a single nucleotide polymorphism (SNP). It will be understood that an individual or population may comprise one or more of the same or different types of mutation as described above.

As used herein, the term "homozygote" refers to an individual cell or plant having the same alleles at one or more or all loci. When the term is used with reference to a specific locus or gene, it means at least that locus or gene has the same alleles. As used herein, the term "homozygous" means a genetic condition existing when identical alleles reside at corresponding loci on homologous chromosomes. As used herein, the term "heterozygote" refers to an individual cell or plant having different alleles at one or more or all loci. When the term is used with reference to a specific locus or gene, it means at least that locus or gene has different alleles. As used herein, the term "heterozygous" means a genetic condition existing when different alleles reside at corresponding loci on homologous chromosomes. In certain embodiments, the genomic region of interest and/or one or more marker(s) as described herein is/are homozygous. In certain embodiments, the genomic region of interest and/or one or more marker(s) as described herein are heterozygous. In certain embodiments, the genomic region of interest allele and/or one or more marker(s) allele(s) as described herein is/are homozygous. In certain embodiments, the genomic region of interest allele and/or one or more marker(s) allele(s) as described herein are heterozygous.

In certain embodiments, the mutation/marker as defined herein is homozygous. Accordingly, in diploid plants the two alleles are identical, in tetraploid plants the four alleles are identical, and in hexaploid plants the six alleles are identical with respect to the mutation or marker. In certain embodiments, the mutation/marker as defined herein is heterozygous. Accordingly, in diploid plants the two alleles are not identical, in tetraploid plants the four alleles are not identical (for instance only one, two, or three alleles comprise the specific mutation/marker), and in hexaploid plants the six alleles are not identical with respect to the mutation or marker (for instance only one, two, three, four or five alleles comprise the specific mutation/marker). Similar considerations apply in case of pseudopolyploid pants.

A "marker" is a (means of finding a) position on a genetic or physical map, or else linkages among markers and trait loci (loci affecting traits). The position that the marker detects may be known via detection of polymorphic alleles and their genetic mapping, or else by hybridization, sequence match or amplification of a sequence that has been physically mapped. A marker can be a DNA marker (detects DNA polymorphisms), a protein (detects variation at an encoded polypeptide), or a simply inherited phenotype (such as the 'waxy' phenotype). A DNA marker can be developed from genomic nucleotide sequence or from expressed nucleotide sequences (e.g., from a spliced RNA or a cDNA). Depending on the DNA marker technology, the marker may consist of complementary primers flanking the locus and/or complementary probes that hybridize to polymorphic alleles at the locus. The term marker locus is the locus (gene, sequence or nucleotide) that the marker detects. "Marker" or "molecular marker" or "marker locus" may also be used to denote a nucleic acid or amino acid sequence that is sufficiently unique to characterize a specific locus on the genome. Any detectable polymorphic trait can be used as a marker so long as it is inherited differentially and exhibits linkage disequilibrium with a phenotypic trait of interest.

Markers that detect genetic polymorphisms between members of a population are well-established in the art. Markers can be defined by the type of polymorphism that they detect and also the marker technology used to detect the polymorphism. Marker types include but are not limited to, e.g., detection of restriction fragment length polymorphisms (RFLP), detection of isozyme markers, randomly amplified polymorphic DNA (RAPD), amplified fragment length polymorphisms (AFLPs), detection of simple sequence repeats (SSRs), detection of amplified variable sequences of the plant genome, detection of self-sustained sequence replication, or detection of single nucleotide polymorphisms (SNPs). SNPs can be detected e.g. via DNA sequencing, PCR-based sequence specific amplification methods, detection of polynucleotide polymorphisms by allele specific hybridization (ASH), dynamic allele-specific hybridization (DASH), molecular beacons, microarray hybridization, oligonucleotide ligase assays, Flap endonucleases, 5' endonucleases, primer extension, single strand conformation polymorphism (SSCP) or temperature gradient gel electrophoresis (TGGE). DNA sequencing, such as the pyrosequencing technology has the advantage of being able to detect a series of linked SNP alleles that constitute a haplotype. Haplotypes tend to be more informative (detect a higher level of polymorphism) than SNPs.

In certain embodiments, the mutation/marker as described herein comprises one or more nucleotide insertions. In certain embodiments, the mutation/marker as described herein comprises one or more nucleotide deletions. In certain embodiments, the mutation/marker as described herein comprises one or more nucleotide exchanges (i.e. A to T, A to G, A to C, T to A, T to C, T to G, C to a, C to T, C to G, G to A, G to T, or G to C exchanges). Depending on the mutagenesis method, certain nucleotide exchanges may be preferred, such as described herein elsewhere for EMS mutagenesis. In certain embodiments, when more than one marker is considered, the separate markers may be of the same or a different type, such as one marker comprising a nucleotide exchange and another marker comprising an insertion. In certain preferred embodiments, the one or more marker each comprises a single nucleotide exchange. In certain preferred embodiments, the one or more marker is a single nucleotide polymorphism (SNP).

As used herein, an "allele" refers to alternative forms of various genetic units associated with different forms of a gene or of any kind of identifiable genetic element, which are alternative in inheritance because they are situated at the same locus in homologous chromosomes. In a diploid cell or organism, the two alleles of a given gene (or marker) typically occupy corresponding loci on a pair of homologous chromosomes.

A "marker allele", alternatively an "allele of a marker locus", can refer to one of a plurality of polymorphic nucleotide sequences found at a marker locus in a population. With regard to a SNP marker, allele refers to the specific nucleotide base present at that SNP locus in that individual plant.

In certain embodiments, the mutation/marker as referred to herein is a particular allele. Accordingly, in certain embodiments, the mutation/marker as referred to herein is a mutation allele or marker allele, i.e. a particular variant of a nucleotide sequence, such as a particular polymorphism.

"Fine-mapping" refers to methods by which the position of a genomic region of interest, such as a QTL, can be determined more accurately (narrowed down) and by which the size of the introgression fragment comprising the QTL is reduced. For example Near Isogenic Lines for the QTL (QTL-NILs) can be made, which contain different, overlapping fragments of the introgression fragment within an otherwise uniform genetic background of the recurrent parent. Such lines can then be used to map on which fragment the genomic region of interest, such as a QTL, is located and to identify a line having a shorter introgression fragment comprising the genomic region of interest, such as a QTL. The random mutagenesis according to the present invention is particularly suitable for fine-mapping, given the more or less regularly interspaced occurrence of introduced mutations.

"Marker assisted selection" (of MAS) is a process by which individual plants are selected based on marker genotypes. "Marker assisted counter-selection" is a process by which marker genotypes are used to identify plants that will not be selected, allowing them to be removed from a breeding program or planting. Marker assisted selection uses the presence of molecular markers, which are genetically linked to a particular locus or to a particular chromosome region (e.g. introgression fragment, transgene, polymorphism, mutation, etc), to select plants for the presence of the specific locus or region (introgression fragment, transgene, polymorphism, mutation, etc). For example, a molecular marker genetically linked to a genomic region of interest as defined herein, can be used to detect and/or select plants comprising the genomic region of interest. The closer the genetic linkage of the molecular marker to the locus (e.g. about 7 cM, 6 cM, 5 cM, 4 cM, 3 cM, 2cM, 1cM, 0.5 cM or less), the less likely it is that the marker is dissociated from the locus through meiotic recombination. Likewise, the closer two markers are linked to each other (e.g. within 7 or 5 cM, 4cM, 3 cM, 2 cM, 1 cM or less) the less likely it is that the two markers will be separated from one another (and the more likely they will co-segregate as a unit). A marker "within 7 cM or within 5 cM, 3 cM, 2 cM, or 1 cM" of another marker refers to a marker which genetically maps to within the 7 cM or 5 cM, 3 cM, 2 cM, or 1 cM region flanking the marker (i.e. either side of the marker). Similarly, a marker within 5 Mb, 3 Mb, 2.5 Mb, 2 Mb, 1 Mb, 0.5 Mb, 0.4 Mb, 0.3 Mb, 0.2 Mb, 0.1 Mb, 50 kb, 20 kb, 10 kb, 5 kb, 2 kb, 1 kb or less of another marker refers to a marker which is physically located within the 5 Mb, 3 Mb, 2.5 Mb, 2 Mb, 1 Mb, 0.5 Mb, 0.4 Mb, 0.3 Mb, 0.2 Mb, 0.1 Mb, 50 kb, 20 kb, 10 kb, 5 kb, 2 kb, 1 kb or less, of the genomic DNA region flanking the marker (i.e. either side of the marker). "LOD-score" (logarithm (base 10) of odds) refers to a statistical test often used for linkage analysis in animal and plant populations. The LOD ("logarithm of odds") score compares the likelihood of obtaining the test data if the two loci (molecular marker loci and/or a phenotypic trait locus) are indeed linked, to the likelihood of observing the same data purely by chance. Positive LOD scores favour the presence of linkage and a LOD score greater than 3.0 is considered evidence for linkage. A LOD score of +3 indicates 1000 to 1 odds that the linkage being observed did not occur by chance.

A "marker haplotype" refers to a combination of alleles at a marker locus.

A "marker locus" is a specific chromosome location in the genome of a species where a specific marker can be found. A marker locus can be used to track the presence of a second linked locus, e.g., one that affects the expression of a phenotypic trait. For example, a marker locus can be used to monitor segregation of alleles at a genetically or physically linked locus.

A "marker probe" is a nucleic acid sequence or molecule that can be used to identify the presence of a marker locus, e.g., a nucleic acid probe that is complementary to a marker locus sequence, through nucleic acid hybridization. Marker probes comprising 30 or more contiguous nucleotides of the marker locus ("all or a portion" of the marker locus sequence) may be used for nucleic acid hybridization. Alternatively, in some aspects, a marker probe refers to a probe of any type that is able to distinguish (i.e., genotype) the particular allele that is present at a marker locus.

The term "molecular marker" may be used to refer to a genetic marker or an encoded product thereof (e.g., a protein) used as a point of reference when identifying a linked locus. A marker can be derived from genomic nucleotide sequences or from expressed nucleotide sequences (e.g., from a spliced RNA, a cDNA, etc.), or from an encoded polypeptide. The term also refers to nucleic acid sequences complementary to or flanking the marker sequences, such as nucleic acids used as probes or primer pairs capable of amplifying the marker sequence. A "molecular marker probe" is a nucleic acid sequence or molecule that can be used to identify the presence of a marker locus, e.g., a nucleic acid probe that is complementary to a marker locus sequence. Alternatively, in some aspects, a marker probe refers to a probe of any type that is able to distinguish (i.e., genotype) the particular allele that is present at a marker locus. Nucleic acids are "complementary" when they specifically hybridize in solution, e.g., according to Watson-Crick base pairing rules. Some of the markers described herein are also referred to as hybridization markers when located on an indel region, such as the non- collinear region described herein. This is because the insertion region is, by definition, a polymorphism vis a vis a plant without the insertion. Thus, the marker need only indicate whether the indel region is present or absent. Any suitable marker detection technology may be used to identify such a hybridization marker, e.g. SNP technology is used in the examples provided herein.

"Genetic markers" are nucleic acids that are polymorphic in a population and where the alleles of which can be detected and distinguished by one or more analytic methods, e.g., RFLP, AFLP, isozyme, SNP, SSR, and the like. The terms "molecular marker" and "genetic marker" are used interchangeably herein. The term also refers to nucleic acid sequences complementary to the genomic sequences, such as nucleic acids used as probes. Markers corresponding to genetic polymorphisms between members of a population can be detected by methods well- established in the art. These include, e.g., PCR-based sequence specific amplification methods, detection of restriction fragment length polymorphisms (RFLP), detection of isozyme markers, detection of polynucleotide polymorphisms by allele specific hybridization (ASH), detection of amplified variable sequences of the plant genome, detection of self-sustained sequence replication, detection of simple sequence repeats (SSRs), detection of single nucleotide polymorphisms (SNPs), or detection of amplified fragment length polymorphisms (AFLPs). Well established methods are also know for the detection of expressed sequence tags (ESTs) and SSR markers derived from EST sequences and randomly amplified polymorphic DNA (RAPD). Without limitation, screening may encompass or comprise sequencing, hybridization based methods (such as (dynamic) allele-specific hybridization, molecular beacons, SNP microarrays), enzyme based methods (such as PCR, KASP (Kompetitive Allele Specific PCR), RFLP, ALFP, RAPD, Flap endonuclease, primer extension, 5'-nuclease, oligonucleotide ligation assay), post-amplification methods based on physical properties of DNA (such as single strand conformation polymorphism, temperature gradient gel electrophoresis, denaturing high performance liquid chromatography, high-resolution melting of the entire amplicon, use of DNA mismatch-binding proteins, SNPlex, surveyor nuclease assay), etc.

A "polymorphism" is a variation in the DNA between two or more individuals within a population. A polymorphism preferably has a frequency of at least 1 % in a population. A useful polymorphism can include a single nucleotide polymorphism (SNP), a simple sequence repeat (SSR), or an insertion/deletion polymorphism, also referred to herein as an "indel". The term "indel" refers to an insertion or deletion, wherein one line may be referred to as having an inserted nucleotide or piece of DNA relative to a second line, or the second line may be referred to as having a deleted nucleotide or piece of DNA relative to the first line.

"Physical distance" between loci (e.g. between molecular markers and/or between phenotypic markers) on the same chromosome is the actually physical distance expressed in bases or base pairs (bp), kilo bases or kilo base pairs (kb) or megabases or mega base pairs (Mb).

"Genetic distance" between loci (e.g. between molecular markers and/or between phenotypic markers) on the same chromosome is measured by frequency of crossing-over, or recombination frequency (RF) and is indicated in centimorgans (cM). One cM corresponds to a recombination frequency of 1%. If no recombinants can be found, the RF is zero and the loci are either extremely close together physically or they are identical. The further apart two loci are, the higher the RF.

A "physical map" of the genome is a map showing the linear order of identifiable landmarks (including genes, markers, etc.) on chromosome DNA. However, in contrast to genetic maps, the distances between landmarks are absolute (for example, measured in base pairs or isolated and overlapping contiguous genetic fragments) and not based on genetic recombination (that can vary in different populations).

An allele "negatively" correlates with a trait when it is linked to it and when presence of the allele is an indicator that a desired trait or trait form will not occur in a plant comprising the allele. An allele "positively" correlates with a trait when it is linked to it and when presence of the allele is an indicator that the desired trait or trait form will occur in a plant comprising the allele.

A centimorgan ("cM") is a unit of measure of recombination frequency. One cM is equal to a 1 % chance that a marker at one genetic locus will be separated from a marker at a second locus due to crossing over in a single generation.

As used herein, the term "chromosomal interval" designates a contiguous linear span of genomic DNA that resides in planta on a single chromosome. The genetic elements or genes located on a single chromosomal interval are physically linked. The size of a chromosomal interval is not particularly limited. In some aspects, the genetic elements located within a single chromosomal interval are genetically linked, typically with a genetic recombination distance of, for example, less than or equal to 20 cM, or alternatively, less than or equal to 10 cM. That is, two genetic elements within a single chromosomal interval undergo recombination at a frequency of less than or equal to 20% or 10%.

The term "linked" or "closely linked", in the present application, means that recombination between two linked loci occurs with a frequency of equal to or less than about 20% (i.e., are separated on a genetic map by not more than 20 cM). Put another way, the closely linked loci co-segregate at least 80% of the time. Marker loci are especially useful with respect to the subject matter of the current disclosure when they demonstrate a significant probability of co-segregation (linkage) with a desired trait. Closely linked loci such as a marker locus and a second locus can display an inter-locus recombination frequency of 20% or less, such as 10% or less, preferably about 9% or less, still more preferably about 8% or less, yet more preferably about 7% or less, still more preferably about 6% or less, yet more preferably about 5% or less, still more preferably about 4% or less, yet more preferably about 3% or less, and still more preferably about 2% or less. In highly preferred embodiments, the relevant loci display a recombination a frequency of about 1 % or less, e.g., about 0.75% or less, more preferably about 0.5% or less, or yet more preferably about 0.25% or less. Two loci that are localized to the same chromosome, and at such a distance that recombination between the two loci occurs at a frequency of less than 20%, such as less than 10% (e.g., about 9 %, 8%, 7%, 6%, 5%, 4%, 3%, 2%, 1 %, 0.75%, 0.5%, 0.25%, or less) are also said to be "proximal to" each other. In some cases, two different markers can have the same genetic map coordinates. In that case, the two markers are in such close proximity to each other that recombination occurs between them with such low frequency that it is undetectable.

"Linkage" refers to the tendency for alleles to segregate together more often than expected by chance if their transmission was independent. Typically, linkage refers to alleles on the same chromosome. Genetic recombination occurs with an assumed random frequency over the entire genome. Genetic maps are constructed by measuring the frequency of recombination between pairs of traits or markers. The closer the traits or markers are to each other on the chromosome, the lower the frequency of recombination, and the greater the degree of linkage. Traits or markers are considered herein to be linked if they generally co- segregate. A 1/100 probability of recombination per generation is defined as a genetic map distance of 1.0 centiMorgan (1.0 cM). The term "linkage disequilibrium" refers to a non-random segregation of genetic loci or traits (or both). In either case, linkage disequilibrium implies that the relevant loci are within sufficient physical proximity along a length of a chromosome so that they segregate together with greater than random (i.e., non-random) frequency. Markers that show linkage disequilibrium are considered linked. Linked loci co-segregate more than 50% of the time, e.g., from about 51 % to about 100% of the time. In other words, two markers that co-segregate have a recombination frequency of less than 50% (and by definition, are separated by less than 50 cM on the same linkage group.) As used herein, linkage can be between two markers, or alternatively between a marker and a locus affecting a phenotype, such as the genomic region of interest as defined herein elsewhere. A marker locus can be "associated with" (linked to) a trait. The degree of linkage of a marker locus and a locus affecting a phenotypic trait is measured, e.g., as a statistical probability of co-segregation of that molecular marker with the phenotype (e.g., an F statistic or LOD score).

The genetic elements or genes located on a single chromosome segment are physically linked. In some embodiments, the two loci are located in close proximity such that recombination between homologous chromosome pairs does not occur between the two loci during meiosis with high frequency, e.g., such that linked loci co-segregate at least about 80% of the time, preferably at least 90% of the time, e.g., 91%, 92%, 93%, 94%, 95%, 96%, 97%, 98%, 99%, 99.5%, 99.75%, or more of the time. The genetic elements located within a chromosomal segment are also "genetically linked", typically within a genetic recombination distance of less than or equal to 50cM, e.g., about 49, 48, 47, 46, 45, 44, 43, 42, 41, 40, 39, 38, 37, 36, 35, 34, 33, 32, 31, 30, 29, 28, 27, 26, 25, 24, 23, 22, 21, 20, 19, 18, 17, 16, 15, 14, 13, 12, 11, 10, 9, 8, 7, 6, 5, 4, 3, 2, 1, 0.75, 0.5, 0.25 cM or less. That is, two genetic elements within a single chromosomal segment undergo recombination during meiosis with each other at a frequency of less than or equal to about 50%, e.g., about 49%, 48%, 47%, 46%, 45%, 44%, 43%, 42%, 41%, 40%, 39%, 38%, 37%, 36%, 35%, 34%, 33%, 32%, 31%, 30%, 29%, 28%, 27%, 26%, 25%, 24%, 23%, 22%, 21%, 20%, 19%, 18%, 17%, 16%, 15%, 14%, 13%, 12%, 11%, 10%, 9%, 8%, 7%, 6%, 5%, 4%, 3%, 2%, 1%, 0.75%, 0.5%, 0.25% or less. "Closely linked" markers display a cross over frequency with a given marker of about 10% or less, e.g., 9%, 8%, 7%, 6%, 5%, 4%, 3%, 2%, 1%, 0.75%, 0.5%, 0.25% or less (the given marker locus is within about 10 cM of a closely linked marker locus, e.g., 9, 8, 7, 6, 5, 4, 3, 2, 1, 0.75, 0.5, 0.25 cM or less of a closely linked marker locus). Put another way, closely linked marker loci co-segregate at least about 80% of the time, such as at least 90% the time, e.g., 91%, 92%, 93%, 94%, 95%, 96%, 97%, 98%, 99%, 99.5%, 99.75%, or more of the time.

As used herein, the term "sequence identity" refers to the degree of identity between any given nucleic acid sequence and a target nucleic acid sequence. Percent sequence identity is calculated by determining the number of matched positions in aligned nucleic acid sequences, dividing the number of matched positions by the total number of aligned nucleotides, and multiplying by 100. A matched position refers to a position in which identical nucleotides occur at the same position in aligned nucleic acid sequences. Percent sequence identity also can be determined for any amino acid sequence. To determine percent sequence identity, a target nucleic acid or amino acid sequence is compared to the identified nucleic acid or amino acid sequence using the BLAST 2 Sequences (BI2seq) program from the stand-alone version of BLASTZ containing BLASTN and BLASTP. This stand-alone version of BLASTZ can be obtained from Fish & Richardson's web site (World Wide Web at fr.com/blast) or the U.S. government's National Center for Biotechnology Information web site (World Wide Web at ncbi.nlm.nih.gov). Instructions explaining how to use the BI2seq program can be found in the readme file accompanying BLASTZ. BI2seq performs a comparison between two sequences using either the BLASTN or BLASTP algorithm.

BLASTN is used to compare nucleic acid sequences, while BLASTP is used to compare amino acid sequences. To compare two nucleic acid sequences, the options are set as follows: -i is set to a file containing the first nucleic acid sequence to be compared (e.g. , C:\seq I .txt); -j is set to a file containing the second nucleic acid sequence to be compared (e.g. , C:\seq2.txt); -p is set to blastn; -o is set to any desired file name (e.g. , C :\output.txt); -q is set to - 1 ; -r is set to 2; and all other options are left at their default setting. The following command will generate an output file containing a comparison between two sequences: C:\B12seq -i c:\seql .txt -j c:\seq2.txt -p blastn -o c:\output.txt -q - 1 -r 2. If the target sequence shares homology with any portion of the identified sequence, then the designated output file will present those regions of homology as aligned sequences. If the target sequence does not share homology with any portion of the identified sequence, then the designated output file will not present aligned sequences. Once aligned, a length is determined by counting the number of consecutive nucleotides from the target sequence presented in alignment with the sequence from the identified sequence starting with any matched position and ending with any other matched position. A matched position is any position where an identical nucleotide is presented in both the target and identified sequences. Gaps presented in the target sequence are not counted since gaps are not nucleotides. Likewise, gaps presented in the identified sequence are not counted since target sequence nucleotides are counted, not nucleotides from the identified sequence. The percent identity over a particular length is determined by counting the number of matched positions over that length and dividing that number by the length followed by multiplying the resulting value by 100. For example, if (i) a 500-base nucleic acid target sequence is compared to a subject nucleic acid sequence, (ii) the BI2seq program presents 200 bases from the target sequence aligned with a region of the subject sequence where the first and last bases of that 200-base region are matches, and (iii) the number of matches over those 200 aligned bases is 180, then the 500-base nucleic acid target sequence contains a length of 200 and a sequence identity over that length of 90% (i.e. , 180 / 200 x 100 = 90). It will be appreciated that different regions within a single nucleic acid target sequence that aligns with an identified sequence can each have their own percent identity. It is noted that the percent identity value is rounded to the nearest tenth. For example, 78.11, 78.12, 78.13, and 78.14 are rounded down to 78.1, while 78.15, 78.16, 78.17, 78.18, and 78.19 are rounded up to 78.2. It also is noted that the length value will always be an integer.

An "isolated nucleic acid sequence" or "isolated DNA" refers to a nucleic acid sequence which is no longer in the natural environment from which it was isolated, e.g. the nucleic acid sequence in a bacterial host cell or in the plant nuclear or plastid genome. When referring to a "sequence" herein, it is understood that the molecule having such a sequence is referred to, e.g. the nucleic acid molecule. A "host cell" or a "recombinant host cell" or "transformed cell" are terms referring to a new individual cell (or organism) arising as a result of at least one nucleic acid molecule, having been introduced into said cell. The host cell is preferably a plant cell or a bacterial cell. The host cell may contain the nucleic acid as an extra-chromosomally (episomal) replicating molecule, or comprises the nucleic acid integrated in the nuclear or plastid genome of the host cell, or as introduced chromosome, e.g. minichromosome.

When reference is made to a nucleic acid sequence (e.g. DNA or genomic DNA) having "substantial sequence identity to" a reference sequence or having a sequence identity of at least 80%>, e.g. at least 85%, 90%, 95%, 98%> or 99%> nucleic acid sequence identity to a reference sequence, in one embodiment said nucleotide sequence is considered substantially identical to the given nucleotide sequence and can be identified using stringent hybridisation conditions. In another embodiment, the nucleic acid sequence comprises one or more mutations compared to the given nucleotide sequence but still can be identified using stringent hybridisation conditions. "Stringent hybridisation conditions" can be used to identify nucleotide sequences, which are substantially identical to a given nucleotide sequence. Stringent conditions are sequence dependent and will be different in different circumstances. Generally, stringent conditions are selected to be about 5°C lower than the thermal melting point (Tm) for the specific sequences at a defined ionic strength and pH. The Tm is the temperature (under defined ionic strength and pH) at which 50% of the target sequence hybridises to a perfectly matched probe. Typically stringent conditions will be chosen in which the salt concentration is about 0.02 molar at pH 7 and the temperature is at least 60°C. Lowering the salt concentration and/or increasing the temperature increases stringency. Stringent conditions for RNA-DNA hybridisations (Northern blots using a probe of e.g. 100 nt) are for example those which include at least one wash in 0.2X SSC at 63°C for 20min, or equivalent conditions. Stringent conditions for DNA-DNA hybridisation (Southern blots using a probe of e.g. 100 nt) are for example those which include at least one wash (usually 2) in 0.2X SSC at a temperature of at least 50°C, usually about 55°C, for 20 min, or equivalent conditions. See also Sambrook et al. (1989) and Sambrook and Russell (2001).

In an aspect, the invention relates to the use of one or more of the (genetic) markers described herein for identifying a plant or plant part, such as a plant or plant part having a genomic region of interest (allele). In an aspect, the invention relates to the use of one or more of the (genetic) markers described herein which are able to detect at least one diagnostic marker allele for identifying a plant or plant part having a genomic region of interest (allele). In an aspect, the invention relates to the detection of one or more of the (genetic) marker alleles described herein for identifying a plant or plant part having a genomic region of interest (allele).

The marker alleles of the invention as described herein may be diagnostic marker alleles which are useable for identifying plants or plant parts having a genomic region of interest (allele).

In an aspect, the invention relates to a (isolated) polynucleic acid comprising a marker allele of the invention, or the complement or the reverse complement of a marker allele of the invention. In certain embodiments, the invention relates to a polynucleic acid comprising at least 10 contiguous nucleotides, preferably at least 15 contiguous nucleotides or at least 20 contiguous nucleotides of a marker allele of the invention, or the complement or the reverse complement of a marker allele of the invention. In certain embodiments, the polynucleic acid is capable of discriminating between a marker allele of the invention and a non-molecular marker allele, such as to specifically hybridise with a marker allele of the invention. In certain embodiments, the polynucleic acid or the complement or reverse complement thereof does not (substantially) hybridise with or bind to (genomic) DNA originating from the wild type plant before being subjected to mutagenesis.

In an aspect, the invention relates to a polynucleic acid capable of specifically hybridizing with a marker allele of the invention, or the complement thereof, or the reverse complement thereof.

In certain embodiments, the polynucleic acid is a primer. In certain embodiments, the polynucleic acid is a probe.

In certain embodiments, the polynucleic acid is an allele specific polynucleic acid, such as an allele specific primer or probe, for instance a KSP primer.

In certain embodiments, the polynucleic acid comprises at least 15 nucleotides, such as 16, 17, 18, 19, 20, 21, 22, 23, 24, or 25 nucleotides, such as at least 30, 35, 40, 45, or 50 nucleotides, such as at least 100, 200, 300, or 500 nucleotides.

It will be understood that "specifically hybridizing" means that the polynucleic acid hybridises with the (molecular) marker allele (such as under stringent hybridisation conditions, as defined herein elsewhere), but does not (substantially) hybridise with a polynucleic acid not comprising the marker allele or is (substantially) incapable of being used as a PCR primer. By means of example, in a suitable readout, the hybridization signal with the marker allele or PCR amplification of the marker allele is at least 5 times, preferably at least 10 times stronger or more than the hybridisation signal with a non-marker allele, or any other sequence.

In an aspect, the invention relates to a kit comprising such polynucleic acids, such as primers (comprising forward and/or reverse primers) and/or probes. The kit may further comprise instructions for use.

In will be understood that in embodiments relating to a set of forward and reverse primers, only one of both primers (forward or reverse) may need to be capable of discriminating between a marker allele of the invention and a non-marker allele, and hence may be unique. The other primer may or may not be capable of discriminating between a marker allele of the invention and a non-marker allele, and hence may be unique.

The aspects and embodiments of the invention are further supported by the following non-limiting examples.

### EXAMPLES

### EXAMPLE 1

If a target is localized in an area of low polymorphism within breeding material, it is very difficult to select for recombination between the target and the flanking region, which is necessary to prevent any linkage drag. In this case, it is helpful to introduce new polymorphisms in close genetic distances to the target, such as for instance 0.1-20 cM on one or both sides of the target. The distribution of these new polymorphisms preferably should be relatively equal.

Random mutagenesis of *Zea mays* was performed using TILLING, which can be done either by chemical treatment or by radiation, which results in new polymorphisms with a distance of approximately 100-200 kb. This corresponds mostly to a genetic distance of less than 1 cM. TILLING (including EMS mutagenesis) was essentially performed as described in Till et al. "Discovery of induced point mutations in maize genes by TILLING"; BMC Plant Biol. 2004 Jul 28;4:12; and Weil & Monde "Getting the point-mutations in maize" Crop Sci 2007; 47 S60-67.

The gene F35H was identified as causative gene of a strong digestibility QTL. This gene is located in an area of very low polymorphism in the iodent pool. A second positive allele of the gene F35H with a similar effect as the original QTL allele could be identified by a reverse TILLING screen of the mutagenized population of PH207. This allele was fixed by selfing and the corresponding line was called PH207m023a (also described in WO2019/206927). A marker (ma61134s06) was developed on the functional polymorphism.

PH207 is a founder of the lodent pool and carries the same fixed region around F35H as the iodent breeding material. PH207m023a carries a positive allele for digestibility and is very similar to iodent breeding material, but carries additional SNPs derived of EMS mutagenesis. In order to identify these SNPs, the line was sequenced by Illumina technology (40x coverage) and the reads were mapped to the PH207 public reference genome (Hirsch, C. N., Hirsch, C. D., Brohammer, A. B., Bowman, M. J., Soifer, I., Barad, O., ... & Fields, C. J. (2016). Draft assembly of elite inbred line PH207 provides insights into genomic and transcriptome diversity in maize. The Plant Cell, 28(11), 2700-2714.). The mapping resulted in an unexpected high number of mostly false positive SNPs, which had to be screened for potential EMS mutations. The screening was done in the following way:
1. The region of interest was determined by introgression of the physical map to a genetic map of the iodent pool and fixed to an area of 20 cM up and downstream of F35H.
2. As the functional SNP was homozygous in PH207m023a, it was supposed, that the whole area was homozygous. Heterozygous SNPs in the area were discarded.
3. EMS causes mostly G->A or C->T mutations. Other SNPs were discarded.
4. The flanking region of the selected SNPs had to be non-repetitive.

In this way, a series of SNPs with an equal distribution over the 40 cM area could be identified, which were successfully converted to KASP markers (Figure 1). These markers are used to prevent any linkage drag when the positive allele of PH207m023 is introgressed in breeding material.

Thereby, a new TILLING line was identified, which had the same positive property as the original QTL line. But the method could also be used in a different way. As described above, in order to introduce SNPs around the gene of interest, a small TILLING population has been set up of a line carrying the QTL of interest. Out of this population, a line was selected, which does not loose the positive property. The line has been fixed by selfing - alternatively haploidization and doubling would have been possible- and sequenced. Flanking SNPs have been identified as described above.

## Claims

1. Method for generating a plant or plant part or for introducing one or more genetic marker linked with a genomic region of interest in a plant or plant part, comprising subjecting a plant or plant part population to random mutagenesis and selecting a plant or plant part or progeny thereof comprising a genetic marker introduced by random mutagenesis and linked with a genomic region of interest.

2. Use of random mutagenesis for introducing one or more genetic marker linked with a genomic region of interest in a plant or plant part population.

3. The method or use according to claim 1 or 2, wherein said genetic marker is unique in said plant or plant part population.

4. The method or use according to any of claims 1 to 3, wherein said one or more genetic marker is flanking said genomic region of interest.

5. The method or use according to any of claims 1 to 4, wherein said one or more genetic marker is located within 20 cM, preferably 10 cM, more preferably 2 cM of said genomic region of interest.

6. The method or use according to any of claims 1 to 5, wherein said one or more genetic marker comprises one or more of one or more insertion, one or more deletion, or one or more SNP.

7. The method or use according to any of claims 1 to 6, wherein said genomic region of interest comprises a locus of a trait of interest.

8. The method or use according to any of claims 1 to 7, wherein said genomic region of interest comprises a gene or a QTL.

9. The method or use according to any of claims 1 to 8, wherein said genomic region of interest is in an area of low polymorphism.

10. The method or use according to any of claims 1 to 9, wherein said one or more genetic marker is not a functional mutation.

11. The method or use according to any of claims 1 to 10, wherein said mutagenesis comprises chemical mutagenesis or irradiation.

12. The method or use according to any of claims 1 to 11, wherein said genetic marker is a genetic marker allele.

13. The method or use according to any of claims 1 to 12, comprising:
- Subjecting a plant or plant part population comprising a genomic region of interest to random mutagenesis;
- Optionally propagating said plant or plant part population and selecting a plant or plant part comprising said genomic region of interest;
- Sequencing at least the 5' and/or 3' flanking region of said genomic region of interest;
- Identifying a plant or plant part comprising one or more mutation introduced in said 5' and/or 3' flanking region.

14. Method for identifying a plant or plant part or one or more genetic marker linked with a genomic region of interest in a plant or plant part, comprising identifying or screening for the presence of one or more genetic marker introduced in a plant or plant part by random mutagenesis and linked with a genomic region of interest.

15. Use of a randomly mutated plant or plant part population, or progeny thereof, for identifying one or more genetic marker introduced by random mutagenesis and linked with a genomic region of interest or for identifying a plant or plant part comprising one or more genetic marker introduced by random mutagenesis and linked with a genomic region of interest.
